(19) Europäisches Patentamt
European Patent Office
Office européen des brevets

(11) Publication number: **0 058 063**
**B1**

(12) # EUROPEAN PATENT SPECIFICATION

(45) Date of publication of patent specification: **23.05.84**

(51) Int. Cl.[3]: **C 07 C103/52**// A23L1/236

(21) Application number: **82300583.0**

(22) Date of filing: **05.02.82**

(54) **Process for removing an n-formyl group.**

(30) Priority: **10.02.81 JP 18588/81**

(43) Date of publication of application: **18.08.82 Bulletin 82/33**

(45) Publication of the grant of the patent: **23.05.84 Bulletin 84/21**

(84) Designated contracting states: **BE CH DE FR GB IT LI NL**

(56) References cited:
**US-A- 3 879 372**
**US-A- 3 933 781**
**US-A- 4 071 511**
**US-A- 4 173 562**

(73) Proprietor: **AJINOMOTO CO., INC.**
**5-8, Kyobashi 1-chome, Chuo-ku**
**Tokyo 104 (JP)**

(72) Inventor: **Ariyoshi, Yasuo**
**No. 20-14, 4 chome, Ooka Minami-ku**
**Yokohama-shi Kanagawa-ken (JP)**
Inventor: **Takemoto, Tadashi**
**No. 1155-2, Nakamaruko Nakahara-ku**
**Kawasaki-shi Kanagawa-ken (JP)**

(74) Representative: **Jones, Michael Raymond et al**
**HASELTINE LAKE & CO. 28 Southampton Buildings**
**Chancery Lane**
**London WC2A 1AT (GB)**

**EP 0 058 063 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to a process for removing an N-formyl group, which may be referred to as " For "hereinafter ; more specifically the present invention relates to a deformylation process comprising removing the formyl group efficiently and selectively from an L-aspartyl-L-phenylalanine lower alkyl ester of which the amino group is protected by a formyl group, i. e. an N-formyl-L-aspartyl-L-phenylalanine lower alkyl ester. The For-protected compound has one or other of the following general formulae, depending on whether the aspartyl moiety is α or β, and may be referred to as " For-APR " hereinafter :

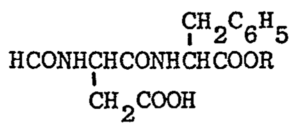
(α)

or

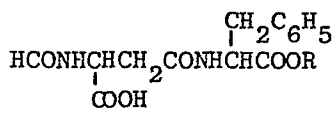
(β)

wherein R represents a lower alkyl radical, preferably one having up to 3 carbon atoms, i. e. a methyl, ethyl, n-propyl or iso-propyl radical.

The present invention provides a useful means of synthesizing dipeptide sweeteners and more specifically an efficient process for removing the For from an N-formyl-α-L-aspartyl-L-phenylalanine lower alkyl ester, which is sometimes referred to as " For-α-APR " hereinafter.

Incidentally, a For-APR may be easily produced by condensation between an N-formyl-L-aspartic acid anhydride and an L-phenylalanine lower alkyl ester (which is sometimes referred to as " PheOR " hereinafter), as an intermediate for synthesis of an α-L-aspartyl-L-phenylalanine lower alkyl ester, which is sometimes referred to as " α-APR " hereinafter (as disclosed in Japanese Patent Application kokai N° 23,001/1977, corresponding to United States Patent N° 4,071,511).

An N-formyl group, i. e. a formyl group protecting an amino group, has heretofore been removed with, for example, the use of dilute hydrochloric acid, hydrogen peroxide (G. Losse et al., Ann. Chem., 636, 140 (1960), hydrazine (P. Lefrancier et al., Bull. Soc. Chim. France, 1965, 3668), aniline (R. Geiger et al., Chem. Ber., 102, 2487 (1969)) or hydroxylamine (R. Geiger ; Chem. Ber., 101, 3386 (1968)), or by catalytic reduction (G. Losse et al., J. Prakt. Chem., 24, 118 (1964)).

Among these processes for deformylation, the one where dilute hydrochloric acid is used is easy to operate as well as inexpensive and, accordingly, a commercially attractive technique. The dilute hydrochloric acid deformylation is usually carried out, as follows. In connection with simple compounds such as N-formyl amino acids, an acidic hydrolysis is effected by heating the compound to be deformylated with dilute hydrochloric acid (V. du Vigneaud et al. ; J. Biol. Chem., 98, 577 (1932)), whereas, in connection with N-formyl peptides, an acidic-alcoholic hydrolysis is effected wherein the compound to be deformylated is allowed to stand in a methanolic hydrochloric acid having a normality of not more than 0.5 at room temperature for 48 hours, the mild conditions being employed to prevent the peptide bond from splitting (J.C. Sheehan et al. ; J. Am. Chem. Soc., 80, 1158 (1958)).

In connection with N-formyl peptides, like a For-α-APR, which have both an ester group and a free carboxyl group in addition to an amino group protected with a formyl group, the ester and peptide bonds are also hydrolyzed if deformylation is carried out by the acidic hydrolysis, whereas the free carboxyl group is esterified if deformylation is carried out by the acidic-alcoholic hydrolysis. The latter hydrolysis is also commercially disadvantageous because of the long period of time required for hydrolytic deformylation, e. g. as long as 48 hours (Japanese patent application kokoku N°. 17,727/1979, corresponding to United States Patent N°. 3,879,372).

The inventors of the present invention have already proposed some improvements in the deformylation whereby such difficulties are overcome to some extent, e. g. the hydroxylamine-strong acid addition salt method (Japanese patent application kokai N°. 68,520/1976, corresponding to United States Patent N°. 4,021,418) and the aqueous organic solvent-strong acid mixture (Japanese patent application kokai N°. 23,001/1977, referred to hereinabove). These known methods are, however, still not as commercially advantageous as desired in that hydroxylamine is required for the former and an organic solvent is required for the latter.

According to the present invention, there is provided a process for removing the N-formyl group from an L-aspartyl-L-phenylalanine lower alkyl ester of which the amino group of the aspartyl moiety is protected by the formyl group, which process comprises heating said ester in a strong acid having a normality in the range from 0.5 to 3 at a temperature in the range from 70 °C to 150 °C.

More particularly, the inventors have found that, if a For-α-APR is heated in a 0.5-3 N strong acid at 70-150 °C, deformylation takes place selectively and is completed in a short period of time such as 15 seconds 60 minutes, with side-reactions such as hydrolysis of the ester or peptide bond being suppressed.

This invention has been made on the basis of these findings. It has been found, as established by the results of their study of the deformylation being carried out under various reaction conditions, that the deformylation is remarkably affected by the acids used, their concentration, the reaction temperature and the reaction period.

In the event that the acid concentration is too low, a long reaction time is required, with the increased likelihood of hydrolysis of the ester group and splitting of the peptide bond taking place to a remarkable extent. On the other hand, in the event that the acid concentration is too high, there is the danger that the peptide bond and the ester group are hydrolyzed as rapidly as the formyl group is removed.

As regards the reaction temperature, too low a temperature needs a long reaction time for deformylation, which will in turn cause the ester group to be hydrolyzed or the peptide bond to split. Thus a prolonged reaction time will not only deformylate a For-α-APR but also hydrolyze it at its ester or peptide bond.

In consideration of these facts, a strong acid concentration range of from 0.5-3 N, a reaction temperature range of from 70 to 150 °C, and a reaction time range of from 15 seconds to 60 minutes are chosen as the preferred reaction conditions.

With the process of the present invention, the hydrolysis can be effected in the absence of any alcoholic reaction medium.

Examples of strong acids which may be employed to carry out the process of the present invention are mineral acids such as hydrochloric acid and sulphuric acid, and organic acids such as benzenesulphonic acid and trichloroacetic acid. Among these acids, hydrochloric and sulphuric acids are preferred because of cost and easy handling.

The amount of acid to be used is not critical.

The greater such amounts are, the more an alkali is ultimately needed to neutralize the acid. In view of this, an acid usually is preferably used in an amount of from 0.5 to 4 times the number of moles of a For-APR to be deformylated.

The stage at which a strong acid is added to the reaction system is not critical either. All the acid may be added at the beginning of the reaction, or the acid may be added intermittently or continuously little by little as the reaction proceeds. A For-APR may of course be added to an acid.

The desired α-APR resulting from deformylation according to the process of the present invention may be easily separated in any appropriate conventional manner once the reaction is completed. Thus, for instance, the reaction mixture may, after an appropriate reaction period, be adjusted in pH to about 4.7 with the use of an alkali such as sodium carbonate and, if necessary, be allowed to stand at a low temperature. Pure α-APR may be collected by filtering the reaction mixture.

In connection with N-formyl-α-L-aspartyl-L-phenylalanine methyl ester, which is sometimes herein referred to as " For-α-AMP ", its deformylated compound, i. e. α-L-aspartyl-L-phenylalanine methyl ester, which is sometimes herein referred to as " α-APM ", is, unlike any other aspartyl dipeptide ester, precipitated as a sparingly soluble hydrohalogenide from a dilute hydrohalogenic acid (Japanese patent application kokoku N°. 41,425/1974, corresponding to United States Patent N°. 3,798,207). Accordingly, when a dilute hydrochloric acid is employed as strong acid in the process of the present invention, the α-APM which is formed is spontaneously separated as its hydrochloride from the reaction mixture. The hydrochloride may be collected by filtration. Addition of α-APM hydrochloride seed crystals to the reaction mixture after the completion of the reaction facilitates crystallization of the hydrochloride. When sulphuric acid is used as the strong acid, the desired compound may be collected as its hydrochloride from the reaction product mixture by adding hydrochloric acid to the reaction mixture after the completion of the reaction. The α-APM hydrochloride thus obtained may be used as a sweetener (Japanese patent application kokai N°. 13,371/1974). It is, however, usually converted to free α-APM by neutralising the hydrochloride with the use of an alkali such as sodium carbonate in an aqueous solvent, and collected as free α-APM crystals.

As is evident from the foregoing, the process of this invention is especially useful in the production of α-APM.

This present invention provides a commercially very useful process for removing the protecting N-formyl group from a particular formyl-protected peptide, because, in accordance with the present invention, the desired deformylated peptide can be produced with the use of inexpensive reagents such as hydrochloric acid, in a short time (such as 15 seconds to 60 minutes) and with the same or better isolation yield as those achieved in accordance with the conventional prior art processes.

This invention will be further illustrated by reference to the following examples. In the examples, reactions at temperatures below the relevant boiling points were carried out at atmospheric pressure, whereas those at temperatures above the relevant boiling points were carried out under sealed conditions.

## Example 1

8 Millilitres of 2 N hydrochloric acid were introduced into a vessel equipped with a reflux condenser, and were refluxed. Then 3.0 g For-α-APM, i. e. N-formyl-α-L-aspartyl-L-phenylalanine methy ester, were added to the boiling hydrochloric acid. After one minute of refluxing, the reaction mixture was cooled by dipping the vessel into iced water.

To the reaction mass was added a small amount (5 mg) of α-APM hydrochloride dihydrate crystals as seed crystals, and crystallization began immediately. The reaction mixture was allowed to stand overnight in a refrigerator. The α-APM hydrochloride dihydrate crystals which had crystallized were collected by

filtration and dried. Yield, 2.01 g. The filtrate was concentrated under reduced pressure, and to the residue were added 3 ml 1 N hydrochloric acid and a small amount (5 mg) of α-APM hydrochloride dihydrate crystals as seed crystals, after which the resulting mixture was allowed to stand overnight in a refrigerator. The α-APM hydrochloride dihydrate crystals which had crystallized were collected by filtration and dried. Yield, 0.13 g.

The collected α-APM hydrochloride dihydrate crystals totalled 2.14 g, which corresponded to a 62.8 % yield on the basis of the For-α-APM starting material.

2.1 Grams of the collected crystals were dissolved in 35 ml water. The resulting solution was adjusted in pH to 4.7 with sodium carbonate and was then allowed to stand for 5 hours in a refrigerator. The α-APM hemihydrate crystals which crystallized out were collected by filtration and dried. Yield, 1.1 g. The filtrate was concentrated to give another 0.3 g crystals.

The α-APM hemihydrate crystals totalled 1.4 g (82.4 % yield on the basis of the α-APM hydrochloride dihydrate). M.p., 237 ~ 238 °C (uncorrected). $[\alpha]_D^{18} = +32.5°$ (c = 1, acetic acid).

Analysis :

For $C_{14}H_{18}O_5N_2 \cdot 1/2\ H_2O$ :

Calcd. : C, 55.44 % ; H, 6.31 % ; N, 9.24 %.

Found. : C, 55.25 % ; H, 6.34 % ; N, 9.28 %.

## Example 2

15 Millilitres of 2 N hydrochloric acid were added to a vessel equipped with a reflux condenser and brought to boiling. A mixture of 4.0 g For-α-APM and 1.0 g N-formyl-β-aspartyl-L-phenylalanine methyl ester, which is herinafter referred to as " For-β-APM ", was added to the boiling hydrochloric acid. After one minute or refluxing, the reaction mixture was cooled by dipping the vessel in iced water.

To the resulting mass was added with a small amount (5 mg) of α-APM hydrochloride dihydrate crystals as seed crystals, and the mixture was allowed to stand overnight in a refrigerator. The α-APM hydrochloride dihydrate crystals which crystallized out were collected by filtration and dried. Yield, 2.65 g. The filtrate was concentrated under reduced pressure, and to the residue were added 5 ml 2 N hydrochloric acid and a small amount (5 mg) of α-APM hydrochloride dihydrate crystals as seed crystals, after which the resulting mixture was allowed to stand overnight in a refrigerator. The α-APM hydrochloride dihydrate crystals which crystallized out were collected by filtration and dried. Yield, 0.25 g. Similar treatment on the filtrate yielded 0.18 g crystals.

The α-APM hydrochloride dihydrate crystals totalled 3.08 g (67.7 % yield on the basis of the For-α-APM).

A 3.0 g portion of the crystals were dissolved in 50 ml water. The resulting solution was adjusted in pH to 4.8 with sodium carbonate and was allowed to stand overnight in a refrigerator. The α-APM hemihydrate crystals which crystallized out were collected by filtration and dried. Yield, 1.4 g. The filtrate was concentrated under reduced pressure to give another 0.2 g crystals.

The α-APM hemihydrate crystals totalled 1.6 g (64.5 % yield on the basis of the α-APM hydrochloride dihydrate). The β-APM produced by deformylation of For-β-APM remained in solution as it is freely soluble in HCl.

## Example 3

20 Millilitres of 0.5 N hydrochloric acid were introduced into a vessel equipped with a reflux condenser and heated to 90 °C. 2.0 Grams For-α-APM were added to the heated hydrochloric acid and kept at 90 °C for 15 minutes. The reaction mixture was then cooled rapidly by dipping the vessel in iced water.

Thereafter, the reaction mixture had added to it 2 ml conc. hydrochloric acid and 5 mg α-APM · HCl · 2 $H_2O$ crystals as seed crystals, and the mixture was allowed to stand overnight in a refrigerator. The α-APM · HCl · 2 $H_2O$ crystals which crystallized out were collected by filtration and dried. Yield, 1.12 g. The filtrate was concentrated under reduced pressure. The residue had added to it 3 ml 1 N hydrochloric acid and 5 mg α-APM · HCl · 2 $H_2O$ crystals as seed crystals, and was allowed to stand overnight. The crystals which crystallized out were collected by filtration and dried. Yield, 0.2 g.

The α-APM hydrochloride dihydrate crystals totalled 1.32 g (58.0 % on the basis of the For-α-APM). M. p., 127 ~ 128°C, parthy melted at 102 ~ 103 °C, both uncorrected.

Analysis :

for $C_{14}H_{18}O_5N_2 \cdot HCl \cdot 2\ H_2O$ :

Calcd. : C, 45.84 % ; H, 6.32 % ; N, 7.64 %.

Found. : C, 46.04 % ; H, 6.33 % ; N, 7.60 %.

## Example 4

One gram of For-α-APM was added to 6 ml boiling 2 N sulphuric acid. After one minute of boiling, the mixture was cooled rapidly, using iced water.

Thereafter, the mixture had added to it 1 ml conc. hydrochloric acid and 5 mg α-APM · HCl · 2 $H_2O$ crystals as seed crystals, amd was then allowed to stand overnight in a refrigerator.

The crystals which has crystallized out were collected by filtration and dried to give 0.62 g α-APM · HCl · 2 $H_2O$. Yield, 54.9 %.

Example 5

15 Millilitres 1 N hydrochloric acid were introduced into a vessel equipped with a reflux condenser and heated to 95 °C. 3.0 Grams For-APM were added to the heated hydrochloric acid and kept at 92 ~ 95 °C for 5 minutes. The reaction mixture was then cooled rapidly by dipping the vessel in iced water.

Thereafter, to the reaction mixture were added 5 mg α-APM · HCl · 2 $H_2O$ as seed crystals, and the seeded mixture was allowed to stand for 3 hours. The resulting α-APM · HCl · 2 $H_2O$ crystals which formed were collected by filtration and dried. Yield, 1.35 g. The filtrate was concentrated under reduced pressure. To the residue were added 3 ml 1 N hydrochloric acid and 5 mg α-APM · HCl · 2 $H_2O$ crystals as seed crystals, and the mixture was allowed to stand overnight in a refrigerator. The resulting crystals were collected by filtration and dried. Yield, 0.75 g.

The α-APM · HCl · 2 $H_2O$ crystals totalled 2.1 g. Yield, 61.6 %.

Examples 6-10

The general procedure of example 4 was repeared 5 times but with slight variations and the results are listed in the following Table 1.

Table 1

| EXAMPLE | Amount of For-α-APM (g) | Hydrochl. Acid Normality (N) | Hydrochl. Acid Amount (ml) | Reaction Temp. (C) | Reaction Time (min.) | Isolation Yield of α-APM.HCl.2$H_2O$ (%) |
|---|---|---|---|---|---|---|
| 6 | 2 | 3 | 6 | 70 | 10 | 50.1 |
| 7 | 3 | 0.5 | 30 | boiled | 4 | 57.2 |
| 8 | 1 | 1 | 5 | boiled | 2 | 65.9 |
| 9 | 3 | 1 | 15 | boiled | 3 | 63.5 |
| 10 | 2 | 3 | 6 | boiled | 1 | 58.4 |

In connection with example 7, 3 ml conc. hydrochloric acid was added to the reaction mixture after the deformylation reaction, whereby crystallization of α-APM · HCl · 2 $H_2O$ was facilitated.

Example 11

In a thin wall SUS tube of 1-millimetre internal diameter and 10-metre length were placed 5 ml 1 N hydrochloric acid and 50 mg For-α-APM. The tube was sealed at its ends and was dipped for 45 seconds in an oil bath maintained at 120 °C. Immediately thereafter, the tube was taken out of the bath and soaked in an ice bath.

After the reaction mixture was cooled enough, it was taken out of the tube and subjected to quantitative analysis with an amino acid analyzer.

It was found that α-APM had been formed in the reaction mixture in a yield of 64.5 % on the basis of the For-α-APM.

Examples 12-14

The general procedure of example 11 was repeated 3 times but with slight variations and the results are listed in the following Table 2. The room temperature was about 20 °C.

(See Table 2, page 6)

Table 2

| EXAMPLE | Amount of For-α-APM (mg) | Hydrochl. Acid Normality (N) | Amount (ml) | Temp of Oil Bath (°C) | Reaction time (sec.) | Yield of α-APM based on For-α-APM (%) |
|---|---|---|---|---|---|---|
| 12 | 50 | 1 | 5 | 140 | 30 | 63.7 |
| 13 | 50 | 0.5 | 5 | 110 | 180 | 65.3 |
| 14 | 50 | 0.5 | 5 | 140 | 120 | 62.4 |

Incidentally, as a control, water was enclosed in a thin wall SUS tube of 1-millimetre internal diameter. The water-containing tube was soaked in an oil bath, first, of 110 °C, then, of 120 °C and last, of 140 °C. Rise in temperature at the centre of the body of water was measured, and was as shown in the sole figure of the accompanying drawing in which the time (seconds) and the temperature (°C) are indicated on the abscissa and the ordinate axes, respectively.

## Claims

1. A process for removing the N-formyl group from an L-aspartyl-L-phenylalanine lower alkyl ester of which the amino group of the aspartyl moiety is protected by the formyl group, which process comprises heating at a temperature in the range from 70 °C to 150 °C said ester in a strong acid having a normality in the range from 0.5 to 3, the lower alkyl radical having not more than 3 carbon atoms.

2. A process according to Claim 1, wherein the heating is effected for a period in the range from 15 seconds to 60 minutes.

3. A process according to any preceding claim, wherein the heating is effected in the absence of any alcoholic reaction medium.

4. A process according to any preceding claim, wherein said ester is N-formyl-α-L-aspartyl-L-phenylalanine lower alkyl ester.

5. A process according to any preceding claim, wherein said ester is N-formyl-α-L-aspartyl-L-phenylalanine methyl ester.

## Ansprüche

1. Verfahren zur Entfernung der N-Formylgruppe aus einem L-Aspartyl-L-phenylalanin-nieder-Alkylester, dessen Aminogruppe im Aspartylrest mit einer Formylgruppe geschützt ist, dadurch gekennzeichnet, daß der Ester in einer starken Säure mit einer Normalität im Bereich von 0,5 bis 3 auf eine Temperatur im Bereich von 70 °C bis 150 °C erhitzt wird, wobei der niedere Alkylrest nicht mehr als drei Kohlenstoffatome aufweist.

2. Verfahren nach Anspruch 1, bei dem das Erhitzen während einer Dauer im Bereich von 15 Sekunden bis 60 Minuten durchgeführt wird.

3. Verfahren nach einem der vorhergehenden Ansprüche, bei dem das Erhitzen in Abwesenheit eines Alkohols als Reaktionsmedium durchgeführt wird.

4. Verfahren nach einem der vorhergehenden Ansprüche, bei dem der Ester N-Formyl-α-L-aspartyl-L-phenylalaninnieder-Alkylester ist.

5. Verfahren nach einem der vorhergehenden Ansprüche, bei dem der Ester N-Formyl-α-L-aspartyl-L-phenylalaninmethylester ist.

## Revendications

1. Un procédé pour éliminer le groupe N-formyle d'un ester alkylique inférieur de L-aspartyl-L-phénylalanine dont le groupe amino du fragment aspartyle est protégé par le groupe formyle, lequel procédé comprend le chauffage à une température dans la gamme de 70 à 150 °C dudit ester avec un acide fort ayant une normalité dans la gamme de 0,5 à 3, le radical alkyle inférieur n'ayant pas plus de 3 atomes de carbone.

2. Un procédé selon la revendication 1, dans lequel le chauffage est effectué pendant une période comprise dans la gamme de 15 secondes à 60 minutes.

3. Un procédé selon l'une quelconque des revendications précédentes, dans lequel le chauffage est effectué en l'absence de tout milieu réactionnel alcoolique.

4. Un procédé selon l'une quelconque des revendications précédentes, dans lequel ledit ester est un ester alkylique inférieur de N-formyl-α-L-aspartyl-L-phénylalanine.

5. Un procédé selon l'une quelconque des revendications précédentes, dans lequel ledit ester est l'ester méthylique de la N-formyl-α-L-aspartyl-L-phénylalanine.

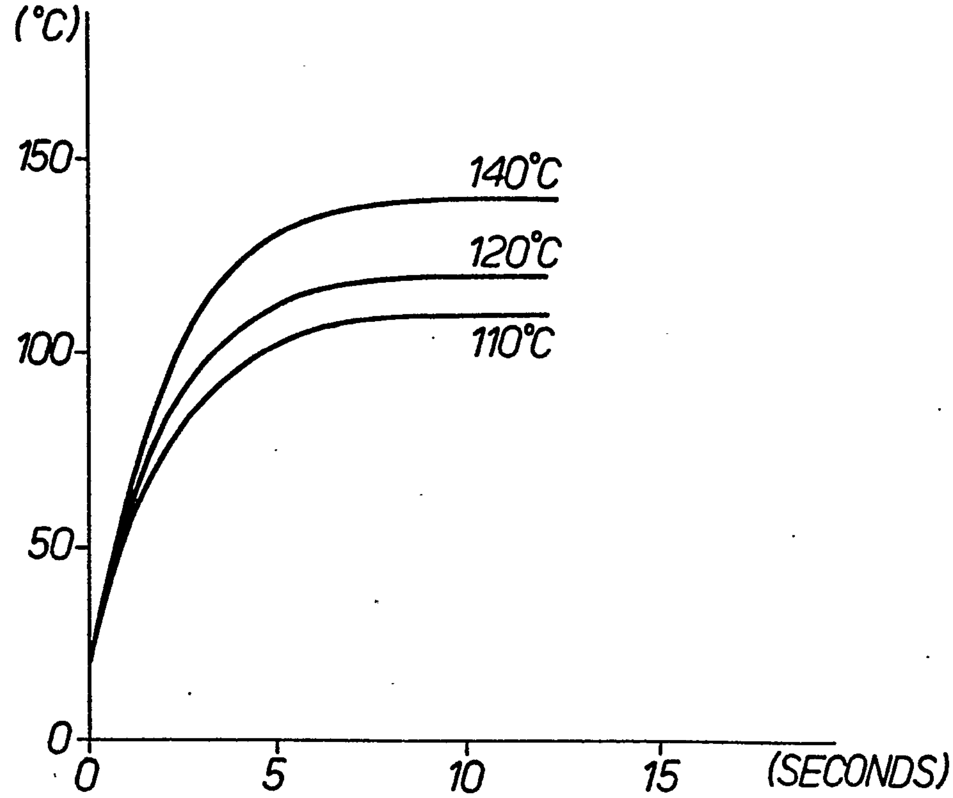
(°C)
150
100
50
0
140°C
120°C
110°C
0
5
10
15
(SECONDS)